## Europäisches Patentamt

## European Patent Office

## Office européen des brevets

(11) Numéro de publication: **0 333 528**
**A1**

# (12) DEMANDE DE BREVET EUROPEEN

(21) Numéro de dépôt: 89400395.3

(22) Date de dépôt: 13.02.89

(51) Int. Cl.⁴: **C 12 N 9/10**
A 23 C 19/032, A 23 L 1/314
// C12N15/00

(30) Priorité: **12.02.88 FR 8801707**

(43) Date de publication de la demande:
**20.09.89 Bulletin 89/38**

(84) Etats contractants désignés:
**AT BE CH DE ES FR GB GR IT LI LU NL SE**

(71) Demandeur: **BONGRAIN S.A.**
**Le Moulin-à-Vent**
**F-78280 Guyancourt (FR)**

(72) Inventeur: **Costes Claude**
**29, rue du Bois du Four**
**78640 Neauphle-le-Château (FR)**

**Frouin André**
**11, rue de Mouchy**
**78000 Versailles (FR)**

(74) Mandataire: **Derambure, Christian et al**
**BUGNION ASSOCIES 55, rue Boissonade**
**F-75014 Paris (FR)**

(54) Utilisation dans les industries alimentaires de micro-organismes exprimant les gènes de transglutaminase pour texturer des mélanges protéiques.

(57) L'invention se rapporte à une nouvelle technologie microbienne de texturation de mélanges protéiques par des micro-organismes exprimant une activité transglutaminase en solution aqueuse.

**EP 0 333 528 A1**

## Description

## UTILISATION DANS LES INDUSTRIES ALIMENTAIRES DE MICRO-ORGANISMES EXPRIMANT LES GENES DE TRANSGLUTAMINASE POUR TEXTURER DES MELANGES PROTEIQUES.

L'object de la présente invention concerne les grandes voies par lesquelles des micro-organismes ayant reçu par manipulations génétiques le ou les gènes d'une transglutaminase peuvent être utilisés dans des fermentations microbiennes exploitables dans les industries alimentaires ou chimiques.

les transglutaminases constituent une classe d'enzymes qui catalysent la réaction des résidus glutamine des protéines avec le groupement aminé, d'une amine ou d'un acide aminé basique comme la lysine. Ces nezymes permettent donc de désamider les protéines riches en glutamine, de greffer la lysine sur une protéine pauvre en cet acide aminé ou de réticuler des protéines diverses entre elles.

Ces enzymes appartiennent au monde des eucaryotes et sont naturellement absentes des procaryotes (bactéries). Elles ont des structures variées et chez les mammifères elles peuvent être cellulaires, dans le foie, les follicules des poils, la prostate, l'utérus, le placenta, ou sanguines dans le plasma et les plaquettes. Dans ce dernier cas une transglutaminase (facteur XIII) intervient dans la coagulation sanguine et le premier usage alimentaire de cet enzyme est sa contribution à la fabrication du boudin.

L'emploi de transglutaminases ajoutées à des protéines sous forme de préparation enzymatique plus ou moins purifiées est in procédé connu. Sur ce sujet la mise au point de G. MATHEIS et J.R. WHITAKER rapporte les travaux antérieurs à 1984 (Journal of Food Biochemistry, 11, 309-327, 1987). Parmi les publications ou brevets, parfois plus récents, parus sur les utilisations potentielles des transglutaminases il y a plusieurs techniques décrites.

La désamidation de certaines protéines (caséines) par l'action d'une transglutaminase avec ou sans acylation préalable accroît la solubilité et les propriétés émulsifiantes des préparations protéiques ( demande de brevet japonais au nom de AJINOMOTO KK - N° 59210097).

Le greffage d'acides aminés variés (lysine, méthionione, cystéine, tryptophane) sur des supports protéiques plus ou moins riches en glutamine par catalyse à l'aide d'une transglutaminase a donné lieu à la prise de deux brevets. Dans les deux cas il s'agit d'accroître les propriétés nutritionnelles de protéines alimentaires (caséine, soja, gluten, viande, poisson, mais, riz) ou de leurs produits de décomposition, et le rôle de l'ion $Ca^{++}$ dans la réaction est souligné ( demande de brevet japonais au nom de SNOW BRAND MILK PRODUCTS N° 58028234 - demande de Brevet japonais au nom de AJINOMOTO N° 61067497).

La fixation d'enzymes ou de coenzymes sur les protéines les plus diverses correspond à quatre brevets.

* Sur des protéines animales, végétales ou bactériennes la fixation du co-enzymes NAD ou NADP à l'aide d'une transglutaminase a été décrite pour des usages pharmaceutiques ou de dépollution (demande de brevet japonais au nom de SNOW BRAND MILK PRODUCTS - N° 58028295).

* Plusieurs types d'enzymes peuvent être immobilisés dans une membrane constituée par réticulation d'une protéine (caséine, autres protéines animale ou végétale) à l'aide d'une transglutaminase. Les enzymes ainsi incluses présentent tous les avantages des catalyseurs fixés sur une membrane (demande de brevet japonais au nom de AJINOMOTO KK - N° 61227783).

* La transglutaminase peut être fixée par l'intermédiaire d'un de ses anticorps monoclonaux luimême lié à une protéine porteuse. Il est ainsi possible de diriger l'activité transglutaminase sur plusieurs cibles spécifiques (demande de brevet japonais au nom de AJINOMOTO KK N° 59175884).

* Deux brevets décrivent la mise en oeuvre des transglutaminases pour préparer des structures particulières. Sur des protéines dispersées en phase liquide (protéines, laitières, collagène, gélatine, protéines de graines végétales) la transglutaminase permet d'obtenir des spécialités cosmétologiques très hydratées, les gels protéiques obtenus étant capables de retenir une importante proportion d'eau (demande de brevet japonais au nom de AJINOMOTO KK N° 61172807). Une variante de cette technique prévoit le moulage du gel protéique, suivi du séchage partiel, ce qui permet de fabriquer des membranes protéiques stables dans l'eau bouillante, insolubles sur une large gamme de pH et d'une résistance mécanique suffisante pour en faire des films d'emballage biodégradables ou des supports de fixation d'enzymes (demande de brevet japonais au nom de AJINOMOTO KK N° 61152247).

* Un usage intéressant la fabrication de spécialités consiste à immobiliser des enzymes utiles dans des préparations alimentaires ou pharmaceutiques par addition de transglutaminase à une protéine pour en faire un réseau gélifié qui inclut ces enzymes. Les protéines mises en oeuvre peuvent être des protéines de soja, ou de la gélatine. Les enzymes citées en exemple sont la glucosidase, la mannosidase, la glucose oxydase, la catalase, l'acide glutamique deshydrogénase et la ribonucléase A (demande de brevet japonais au nom de AJINOMOTO KK N° 59066886).

* Enfin deux brevets concernent directement l'industrie alimentaire. L'un décrit un procédé d'obtention de gels protéiques pour préparations alimentaires réalisées par addition de transglutaminase à des émulsions contenant des protéines, de l'huile ou une graisse et de l'eau (demande de brevet japonais au nom de AJINOMOTO KK N° 59066886). L'autre, premier déposé sur ce thème, décrit la préparation de gels protéiques par réticulation de protéines en solution sous l'action d'une transglutaminase (demande de brevet japonais AJINOMOTO KK N° 58149645).

Outre ces brevets, plusieurs publications scientifiques rapportent des travaux réalisés en collaboration avec AJINOMOTO KK sur l'utilisation des transglutaminases. Ces travaux recoupent exactement le contenu des brevets déposés préalablement et sus-cités. Aucun de ces documents ne rapporte ou évoque la présente invention, notamment la mise en oeuvre de micro-organismes générant une transglutaminase in situ afin de provoquer une texturation des protéines n'est pas rapportée.

L'originalité de l'invention objet de la présente demande, par rapport aux découvertes antérieures et aux brevets précités, consiste à ne pas apporter une préparation enzymatique de transglutaminases objet de tous les brevets cités, mais un germe qui, par suite de modification génétique, est capable de produire ces enzymes en cultivant dans le mélange ou le produit à transformer.

Ce procédé est beaucoup plus économique, puisqu'il élimine toutes les opérations de production et de séparation de l'enzyme antérieures à son emploi.

Ce procédé est aussi beaucoup plus souple, puisqu'il permet de retarder et moduler la production d'enzyme in situ en jouant sur les conditions physicochimiques qui permettent l'expression du gène, pour en obtenir l'effet au moment optimal, et, en fonction de son action, d'arrêter cette production à l'instant désiré lorsque le résultat souhaité est obtenu. Il permet donc de compenser les varations de réactions des matières premières, notamment d'origine agricole, au vu des résultats, en modulant la quantité d'enzymes produites. Ce qui est impossible par les techniques d'addition enzymatique découvertes antérieurement au moins lorsque l'enzyme a pour effet de solidifier le milieu réactionnel.

Ce procédé permet aussi, par le choix judicieux de la souche modifiée, d'obtenir simultanément plusieurs effets, par exemple de combiner une texturation avec une acidification grâce à des bactéries lactiques, ou de combiner une production de gaz ($CO_2$) avec une texturation pour obtenir un produit aéré nouveau, par l'emploi de levures.

Une variante du procédé selon l'invention consiste à précultiver le germe en milieu liquide, puis à concentrer la culture par ultrafiltration, osmose inverse ou lyophilisation avant de l'utiliser, de façon à limiter le temps de culture pour éviter le développement éventuel de germes pathogènes.

Après que l'information génétique des enzymes nommées transglutaminase ait été introduite dans des micro-organismes, l'invention consiste à utiliser ces micro-organismes, transformés par manipulation génétique, dans des mélanges de différentes protéines, avec ou sans amines, et contenant des glucides, pour réaliser plusieurs modes de texturation des protéines au cours d'une fermentation.

L'enzyme dont le gène a été cloné dans les micro-organismes peut avoir plusieurs origines : transglutaminases (appelées TGases) de foie de cobaye ou de lapin, de placenta ou de plaquettes sanguines. La TGase considérée peut avoir une ou plusieurs sous-unités. La transformation génétique des micro-organismes utilisables dans la présente invention est réalisable par les techniques classiques du génie génetique par exemple en utilisant une banque de cDNA ou un RNA messager isolé d'un organe animal comme source de gène, multiplication du gène via un micro-organisme intermédiaire (par exemple Escherichia coli), et introduction du gène dans le micro-organisme cible à l'aide d'un vecteur approprié. Une étape partielle de ce travail est décrite par K. IKURA, T. NASU, H. YOKOTA, R. SASAKI et H. CHIBA (Cloning of cDNA coding for Guinea Pig liver transglutaminase - Agric. biol. Chem., 51, (3), 957-961, 1987). Le gène dont 60% a été cloné sera alors exprimable dans le micro-organisme et la TGase pourra être excrétée ou fixée à la surface de la paroi du micro-organisme : les cellules de ce micro-organisme en suspension en milieu aqueux auront donc à la fois une activité TGase et une propriété fermentaire, mais, dans ce travail japonais, le micro-organisme transformé est Escherichia coli, non utilisable dans l'industrie alimentaire.

Depuis, d'autres micro-organismes ont reçu, par génie génétique, le gène du Facteur XIII, tranglutaminase du plasma sanguin (cf demande de brevet européen au nom de ZYMOGENETICS, Inc. N° 0268772). Les micro-organismes qui permettent la production du Facteur XIII$_A$ sont des souches de Bactéries du genre Escherichia coli, ou divers Bacillus, et également la Levure Saccharomyces cerevisiae ; la Levure Schizosaccharomyces pombe. Les Champignons filamenteux des genres Aspergillus ou Neurospora sont également évoqués. Les micro-organismes créés par ZYMOGENETICS Inc. sont déposés à l'American Type Culture Collection sous les numéros 40260 et 40261. Ainsi, l'une de ces souches, par exemple, pourrait être utilisée dans les technologies qui caractérisent la présente invention.

Notre invention vise à mettre en oeuvre industriellement des micro-organismes producteurs de diverses transglutaminases pour fabriquer des produits alimentaires. Ces nouveaux micro-organismes peuvent donc être, après transformations par les techniques du génie génétique, différentes souches de Levures : Saccharomyces cerevisiae apte à fermenter des solutions contenant du glucose et/ou du saccharose, Kluyveromyces lactis apte à fermenter des solutions de lactose et d'autres glucides, Yarrowia lipolytica apte à se développer sur des solutions protéiques.

Les micro-organismes transformés peuvent être également des bactéries utilisables dans les industries alimentaires comme Bacillus subtilis ou déjà mises en oeuvre comme les bactéries lactiques : Streptococcus lactis, Lactobacillus casei, Lactobacillus lactis, Lactobacillus plantarum. Ces bactéries lactiques transformées ayant conservé leur aptitude à fermenter le lactose du lait peuvent générer de l'acide lactique tout en exprimant une activité TGase.

L'invention consiste à mettre une souche ou plusieurs souches de ces micro-organismes transformés génétiquement dans des mélanges constitués par des solutions artificielles de protéines contenant ou non un glucide fermentescible, et renfermant ou non l'ion $Ca^{++}$ (qui stabilise l'enzyme TGase), ou dans des mélanges naturels comme le lait, les purées de viande, les pâtes et bouillies céréalières, ou dans des mélanges mixtes.

La fermentation peut être conduite à une température convenant au germe modifié : généralement elle se situera entre 10°C et 40°C pour obtenir une action convenable de la transglutaminase.

Il en est de même du pH, dont la zone d'activité de la transglutaminase se situe entre 4 et 10 et l'optimum entre 6 et 8,3.

Les protéines en solution, seules ou en mélange, peuvent être dénaturées par un traitement thermique préalable à l'addition des micro-organismes et au démarrage de la fermentation. Ce traitement dénaturant peut être nécessaire pour rendre certaines protéines aptes à réagir avec la TGase.

La conduite de la fermentation détermine à chaque instant la concentration de l'enzyme TGase active dans la solution : cette conduite doit être pilotée par le réglage de la température, et si le micro-organisme est acidifiant, par le réglage du pH. La concentration initiale en glucides dans la solution est l'un des moyens de régler la variation du pH, donc l'activité TGase.

Le processus peut être arrêté par un nouveau traitement thermique à une température égale ou supérieure à 65°C (temperature de la dénaturation de l'enzyme).

Dans ces conditions la plupart des chaînes polypeptidiques contenant des résidus glutaminyle peuvent être désamidées, ou réticulées, ou désamidées et réticulées. Sie le mélange contient une acylamine lipophile, les protéines peuvent être acylées et acquérir ainsi des propriétés émulsionnantes et/ou moussantes. La réticulation conduit à des gels hydrophiles, bases de nouvelles textures. En pratique toutes les protéines alimentaires d'origine animale, végétale ou microbienne peuvent être transformées par cette nouvelle technologie microbienne de texturation.

EXEMPLES

Exemple 1 : Structuration de viande hachée:

On utilise un lactobacillus plantarum génétiquement modifié pour produire une transglutaminase, de façon à obtenir avec de la viande hachée une pièce de viande ayant une texture aussi cohésive qu'un muscle entier, donc se prêtant au tranchage, avant et après cuisson, de façon similaire aux pièces de viandes non broyées, et à cuire de la même manière.

a) 100kg de viande de boeuf refroidie à 3°C contenant 10° de lipide, sont hachés au broyeur à la grille de 3mm ; cette viande est transvasée dans un pétrin et on lui ajoute 0,5kg de sucre, 500ml de culture de 24h de Lactobacillus plantarum génétiquement modifié à $10^9$ germes/ml. Une fois bien homogénéisée, cette pâte est transférée dans un poussoir sous vide doseur, conditionnée sous vide en sacs plastiques par portions de 100g. Les sachets sont scellés sous vide, puis étuvés 10h à 20°C, puis 15h à 15°C avant d'être congelés. On obtient un steack de texture comparable à un beefsteack découpé dans un muscle.

b) Variante du précédent

100kg de boeuf, 0,5kg de sucre, 500ml de culture sont hachés et homogénéisés au cutter, poussés en pains cylindriques de 1kg, de diamètre 80mm ; ils subissent le même étuvage et sont, de même, congelés. On obtient l'équivalent d'un rôti de boeuf de 1kg avec une texture et des capacités à être tranché comparables.

Exemple 2 : Rôti précuit:

100kg de viande de boeuf sont hachés à 3mm, cuits au four à micro-ondes de façon à porter la température à 80°C pendant 2 minutes, essorés, puis additionnés de 0,4kg de sucre, de 500ml de la culture de lactobacillus plantarum précédemment décrite, poussés en sachets plastiques et étuvés comme dans les exemples précédents. Ce procédé permet d'éliminer les éventuels germes pathogènes résistant à l'acidification provoquée par Lactobacillus plantarum modifié génétiquement.

Exemple 3:

Même procédé que ceux antérieurement cités mais où les 500ml de culture de Lactobacillus plantarum sont remplacés par 50ml d'une culture du même germe préconcentré 100 fois par centrifugation et l'étuvage est réduit à 10h à 20°C.

Exemple 4:

Prendre 100kg de rétendat concentré 5 fois, délactosé par dialyse, à pH 6,4 (rétentat doux), y ajouter 100l d'eau, 1l de culture à $10^8$g/ml de Saccharomyces cerevisiae modifié génétiquement, 20kg de sucre, et de l'arôme de fraise (Firmemich) pour obtenir le goût désiré on mélange soigneusement, on coule 40g en coupelles plastiques rectangulaires de $100^3$, on étuve 15h à 20°C, on colore le dessus au rocou, on chauffe à 75°C 1 minute, et on thermoscelle. On obtient un produit ressemblant à un pain, tranchable, très riche en protéines.

Exemple 5:

Mélanger 100g de protéines de soja, 100g de caséinate haute viscosité, 1000g d'eau, 20g de sel, du carmin de cochenille, des épices au goût souhaité, 10g de sucre, 100g de crème et 1% de culture de Kluyveromyces lactis modifié génétiquement. Brasser fortement sous vide, couler en pains de 1kg, porter 24h à 35°C puis cuire 1h30 à 90°C en étuve humide après avoir emballé sous plastique sous vide. On obtient un pain de simili viande.

Exemple 6:

Même procédé qu'à l'exemple où les protéines de soja sont remplacées par une poudre de gluten, additionnée de lysine ce qui permet de rééquilibrer cette protéine en même temps que de la texturer. Mélanger 200g de gluten, 10g de lysine, 1000g d'eau, 20g de sel, 2g de poivre, 5g d'ail, 0,2g de muscade, 0,1ml de solution de carmin de cochenille, 10g de sucre, 100g de saindoux, 100g de culture de Kluyveromyces lactis modifié génétiquement, bien mélanger, couler en pains de 1g sous gaine de plastique de diamètre 80mm, porter 24h à 35°C puis cuire 1h30 à 85°C en étuve humide. On obtient un pain rose semblable à un mortadelle.

Exemple 7:

Prendre 60kg de rétentat doux, pH6,4, concentration 3, délactosé par rinçages successifs à 0,5% de lactose, y ajouter 40kg de crème douce à 40% de matière grasse, ajouter 1kg de lactate de sodium, 1,5kg de sel, ajouter 1kg de culture de Kluyveromyces lactis génétiquement modifié, ajouter 1kg de concentrat de culture de Brevibacterium linens obtenu sur lait et concentré 5 fois par osmose inverse ; bien mélanger, couler en forme de Pont l'Evêque, étuver 10h à 35°C, porter 2mm à 75°C au micro-ondes, refroidir, colorer la surface avec une solution de rocou, ensemencer avec B. linens et porter 8 jours en haloir selon les conditions opératoires fromagères ordinaires, emballer et livrer comme un Pont l'Evêque ordinaire. On obtient un fromage avec un rendement double de l'ordinaire.

Ces exemples ne sont pas limitatifs, ils sont donnés à titre d'illustration de l'intérêt de notre invention, et du progrès considérable qu'elle présente par rapport à la production et la séparation de l'enzyme avant son éventuel usage. Ils montrent en même temps l'intérêt des fermentations à double but.

**Revendications**

1) Procédé de fermentation microbienne utilisable dans les industries alimentaires ou chimiques à l'aide de micro-organismes transformés par manipulation génétique avec pour effet la production de transglutaminases fonctionnelles in situ.

2) Procédé selon la revendication 1, caractérisé en ce que l'on met au moins une souche de micro-organismes transformés par manipulation génétique et clonage d'un gêne d'enzyme transglutaminase dans des protéines en présence de glucides assimilables.

3) Procédé selon l'une quelconque des revendications 1, 2, caractérisé en ce que l'on cultive les micro-organismes à des températures variant de 5°C à 50°C, à des pH supérieurs à 4 et inférieurs ou égaux à 10 et en réglant la concentration en glucides du milieu.

4) Procédé selon l'une quelconque des revendications 1 à 3, caractérisé en ce que l'on traite thermiquement les micro-organismes à une température égale ou supérieure à 65°C pour arrêter l'activité transglutaminase.

5) Procédé selon l'une quelconque des revendications 1 à 4, caractérisé en ce que l'on ajoute un sel de calcium à la solution en fermentation afin de stabiliser et d'activer l'enzyme transglutaminase au fur et à mesure de sa production microbienne dans le milieu de culture.

6) Procédé selon l'une quelconque des revendications 1 à 4, qui consiste à apporter la ou les souches microbiennes sous forme concentrée, notamment par ultrafiltration, osmose inverse ou lyophilisation.

7) Procédé selon l'une quelconque des revendications 1 à 6, caractérisé en ce que la fonction des transglutaminases produites in situ consiste en une modification de la texture des protéines seules ou associées à d'autres ingrédients alimentaires.

8) Procédé selon l'une quelconque des revendications 1 à 6, caractérisé en ce que la fonction des transglutaminases produites in situ consiste en la texturation de solutions ou suspensions de protéines variées.

9) Procédé selon l'une quelconque des revendications 1 à 6, caractérisé en ce que la fonction des transglutaminases produites in situ consiste en une modification de la valeur alimentaire des protéines.

10) Procédé seln l'une quelconque des revendications 1 à 6, caractérisé en ce que la fonction des transglutaminases produites in situ consiste en un greffage sur les protéines d'enzymes ou de co-enzymes par fermentation microbienne à l'aide de micro-organismes transformés.

11) Produits obtenus par le procédé de l'une quelconque des revendications 1 à 10.

12) Utilisation des produits selon la revendication 11 pour la réalisation de produits de boucherie.

13) Utilisation des produits selon la revendication 11 pour la réalisation de produits de fromagerie.

14) Utilisation des produits selon la revendication 11 pour la réalisation de produits de charcuterie.

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| Y,D | AGRICULTURAL AND BIOLOGICAL CHEMISTRY, vol. 51, no. 3, mars 1987, pages 957-961, Tokyo, JP; K. IKURA: "Cloning of cDNA coding for guinea pig liver transglutaminase" * En entier * --- | 1,2,7,8 ,11 | C 12 N 9/10 A 23 C 19/032 A 23 L 1/314// C 12 N 15/00 |
| Y,D | JOURNAL OF FOOD CHEMISTRY, vol. 11, no. 4, 1987, pages 309-327, Westport, US; G. MATHEIS et al.: "A review: Enzymatic cross-linking of proteins applicable to foods" * Pages 311-312 * ----- | 1,2,7,8 ,11 | |

| DOMAINES TECHNIQUES RECHERCHES (Int. Cl.4) |
|---|
| C 12 N A 23 L A 23 C |

Le présent rapport a été établi pour toutes les revendications

| Lieu de la recherche | Date d'achèvement de la recherche | Examinateur |
|---|---|---|
| LA HAYE | 01-06-1989 | VAN PUTTEN A.J. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
........................................................................
& : membre de la même famille, document correspondant

EPO FORM 1503 03.82 (P0402)